# EUROPEAN PATENT APPLICATION

(11) **EP 2 361 548 A1**
(43) Date of publication of application: **31.08.2011**
(21) Application number: 11250167.1
(22) Date of filing: 14.02.2011
(51) Int. Cl.: A61B 3/107, A61B 3/12, A61B 3/135

(54) **Corneal topography measuring method and corneal topography measurement apparatus**

(30) Priority: 18.02.2010 JP 2010034111
(71) Applicant: TOMEY CORPORATION, Nagoya-shi, Aichi 451-0051 (JP)
(72) Inventor: Oba, Atsuya, Nagoya-shi Aichi 451-0051 (JP); Yamagishi, Koji, Nagoya-shi Aichi 451-0051 (JP)
(74) Representative: Jackson, Martin Peter

(57) **Abstract**

A corneal topography measuring method wherein a plurality of concentric ring lights are projected on a cornea to obtain ring images, and a corneal surface topography is measured by obtaining curvatures of the cornea at respective positions where the ring lights are projected based on positional information of the ring images, the method being characterized by comprising the following steps: judging a necessity for any correction to measured positions of the ring images obtained by projecting the ring lights onto the cornea based on a prescribed correction criteria; determining virtual positions of the ring images based on cross-sectional images of the cornea taken by irradiating slit light onto the cornea; and making a correction to the measured positions of the ring images based on the virtual positions thereof when the correction is deemed necessary to the measured positions of the ring images. A corneal topography measurement apparatus is also disclosed.

## Description

### BACKGROUND OF THE INVENTION

### 1. Field of the Invention

This invention relates to a method and an apparatus for measuring the surface shape (topography) of the cornea of the eye, especially for measuring corneal topography by means of using images obtained by projecting concentric ring lights called "Placido ring" or "Kerato ring" onto the cornea.

### 2. Description of the Related Art

Conventionally, measurement results of corneal topography have been used in examining eyes after surgery, inspecting diseases such as keratoconus, or prescribing contact lenses. As one of the instruments for measuring corneal topography, a measurement apparatus that uses ring images (of Placido ring) obtained by projecting concentric ring lights onto the cornea is used. For example, the one described in Japanese Published Unexamined Application No. JP-A-62-34526 (Patent Literature 1) is such an apparatus.

Meanwhile, the ring image described above appears at different distances from the optical axis depending on the corneal curvature radius at the point where the ring light is projected. Therefore, analyses of those ring images allow us to obtain information about curvature distribution on the corneal surface.

However, using an apparatus with conventional structure for measuring corneal topography, there have been some difficulties in determining how many ring images precede the detected ring image counting from the optical axis of the projected concentric ring light. For example, when detecting a ring image formed by the third ring light from the optical axis, if such ring image is mistaken for the one formed by the second ring light, the measurement result of curvature ends up being outputted as a far smaller diopter value than the real one, whereas if it is mistaken for the one formed by the fourth ring light, the result is outputted as a much larger diopter value than the real one, which can lead to a significant measurement error. Generally, since each detected ring image can be related to a projected ring light in the sequential order from the proximal side of the optical axis, once an error is made in relating a ring image to a ring light, relating of all other ring images in the outer periphery beyond the area goes awry, resulting in a problem of not being able to achieve satisfactory measurement accuracy over a wide area of the corneal surface.

Since the measurement results tend to be highly inaccurate especially in case of cornea with irregular surface, there was an ironic situation where the more necessary the measurement of corneal topography is, the more significant the measurement error is likely to be. In other words, while the corneal surface is covered with tears, corneas with irregular surface tend to have a wide variation in the thickness of the tear film. For that reason, it may possibly occur that the ring images partially disappear or adjacent ring images get too close to or too far from each other or even overlap as a result of deflection of the projected ring light under different thickness of the tear film. As a result, accuracy in correlating a ring image to a projected ring light tends to be compromised, and thus producing significant errors in measuring the curvature of the corneal surface.

In order to deal with these problems, Japanese Published Unexamined Application No. JP-A-2006-158749 (Patent Literature 2) proposes a way to obtain related ring images by means of projecting each of multiple concentric ring lights in sequence onto the cornea. However, it takes fair amount of time to obtain ring images one by one through sequentially projecting each of 10 or more ring lights, which imposes substantial burden on the examinee and examiner requiring too much of their effort. Furthermore, even if the ring lights are projected one by one, it cannot be an effective remedy against the partial disappearance or abnormal displacement of produced ring images due to the variance in the thickness of the tear film and the existence of ambient light and so forth.

### SUMMARY OF THE INVENTION

It is therefore an object of the present invention to provide a new corneal topography measurement method and a corneal topography measurement apparatus capable of measuring corneal topography with high precision by means of using ring images obtained by projecting concentric ring lights onto the cornea.

One aspect of the present invention provides a corneal topography measuring method wherein a plurality of concentric ring lights are projected on a cornea to obtain a plurality of ring images, and a corneal surface topography is measured by obtaining curvatures of the cornea at respective positions where the ring lights are projected based on positional information of the ring images, the method being characterized by comprising the following steps: judging a necessity for any correction to measured positions of the ring images obtained by projecting the ring lights onto the cornea based on a prescribed correction criteria; determining virtual positions of the ring images based on cross-sectional images of the cornea taken by means of irradiating slit light onto the cornea; and making a correction to the measured positions of the ring images based on the virtual positions thereof when the correction is deemed necessary to the measured positions of the ring images.

The method of this invention makes it possible to measure the curvature at each position of the corneal surface with high precision and speed. The reason why this invention achieves such a technical effect is that one of the major characteristics thereof is "the adoption of measured positions of the ring images obtained by projecting multiple concentric ring lights onto the cornea as the basic information, to which corrections are made according to virtual positions of the ring images determined on a basis of the cross-sectional images thereof."

In other words, in addition to "A Method for Measuring Surface Curvature Based on the Positional Information of Ring Images Obtained by Means of Projecting Multiple Concentric Ring Lights Onto the Cornea" as has been presented in JP-A-62-34526 and JP-A-2006-158749, a proposal is made in recent years in "A Method for Determining Surface Curvature Based on Cross-sectional Images of the Cornea Taken by Means of Irradiating Slit Light onto the Cornea" as described in Japanese Published Unexamined Application No. JP-A-63-197432. Under these circumstances, this invention tries to determine the curvature of corneal surface by adopting measured positions of the ring image of the former as the basic information. And the curvature of corneal surface determined on a basis of cross-sectional images of the cornea of the latter is used to virtualize the position of the ring image without adopting itself as a result (curvature of corneal surface to be determined) in order to correct measured positions of the ring image, which is the basic information, by means of using the virtual position of said ring image.

As a result, in measuring the topography (curvature) of corneal surface, it was made possible to effectively avoid the above-mentioned "occurrence of errors in relating a ring image to a projected ring light," which is a problem of a measurement method based on measured positions of the ring image, while keeping inherently higher precision than measuring cross-sectional images of the cornea.

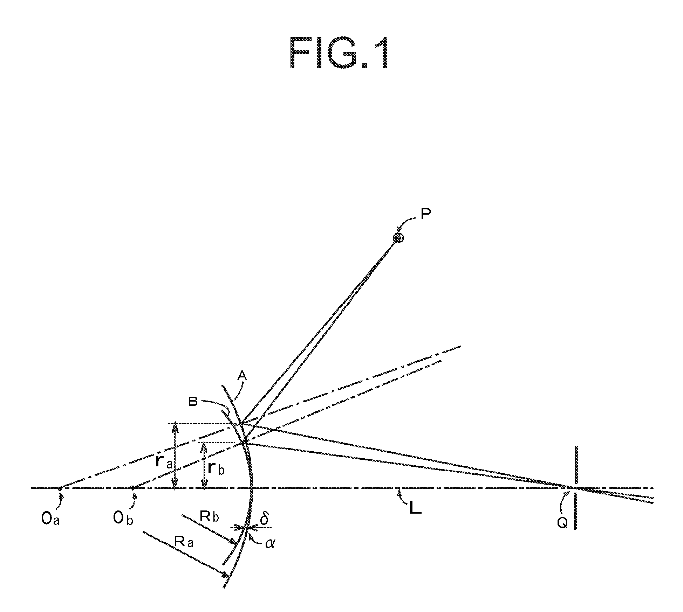

The fact that measured positions of the ring image can contribute to higher precision in measuring curvature than does the cross-sectional image of the cornea obtained by irradiating slit light is understandable from, for example, the cross-sectional diagram in FIG.1. That is, in case of Cornea A with a radius of surface curvature Rₐ and Cornea B with a radius of surface curvature R_{b}, a difference in radius rₐ- r_{b} is observed regarding the ring image, which is a reflected image at the corneal surface of the ring light irradiated from a light source P. Here, Oₐ and O_{b} in FIG. 1 represent the center of curvature of Cornea A and B, respectively. Q represents an optical center (aperture) of the imaging apparatus. In summary, since the apparatus uses reflection of light, the direction of reflected light varies widely due to the slightest change in the tilt of the corneal surface, which is the reflective plane, and utilizing those measured positions of the ring image corresponding to various directions of the reflected light allows us to determine the curvature, with high precision, from the tilting angle of the corneal surface at the point of said reflection.

Meanwhile, even in case of cross-sectional images of the cornea obtained by imaging corneal topography by directly receiving scattered slit light, for example, Corneas A and B with a difference in curvature radius rₐ- r_{b}, the positional difference δ recognizable at their common point α is quite small. In addition, this difference ð totally depends on the resolution of the imaging apparatus CCD and the like. Therefore, it is hard to achieve desired precision around the corneal center that especially requires high precision of measurement.

However, the fact remains that cross-sectional images of the cornea obtained by directly imaging such corneal topography still capture the real shape. For that reason, we focused on the reality that those images have no crucial errors even though they are no match for the values based on measured positions in terms of the precision of curvature measurement. In other words, the corneal curvature determined on a basis of measured positions of the ring image has a problem of "compromised accuracy in correlating a ring image to a projected ring light," although it achieves theoretically high precision, and once such compromise occurs, an extremely significant error is inevitable in measuring corneal topography because the position of light source P itself as shown in FIG. 1 is erroneous. It is then considered that the measured position of the ring image when the compromised accuracy occurs in the above-mentioned correlation produce even more significant errors than the virtual position of the ring image determined on a basis of the cross-sectional image of the cornea.

Therefore, this invention adopts a high-precision measurement method based on measured positions of the ring image in measuring corneal topography (curvature), whereas, if any measurement contains a significant error, it is corrected based on the "virtual positions of the ring image determined on a basis of cross-sectional images of the cornea" that have smaller margin of error than measured positions of the ring image when "accuracy in correlating a ring image to a projected ring light is compromised," thus effectively avoiding any significant error caused by correlating a ring image to a ring light and the like while essentially maintaining high precision achieved on a basis of measured positions of the ring image.

In this invention, the "correction criteria" adopted in the "judgment of a necessity for any correction" is not limited to particular ones as long as they can provide an objective index as to the reliability of measured positions of the ring image. More specifically, this invention can either use judgment criteria based on "whether the measured position of the target ring image is too far off from the virtual position of the corresponding ring image determined from the cross-sectional image of the cornea," or "whether the value of separation distance between the target ring image and its adjacent ring image is too far off from the average value of separation distances between other ring images," or "whether the corneal curvature determined on a basis of measured positions on the target ring image is too far off from the one determined on a basis of the virtual position of the corresponding ring image obtained from the cross-sectional image of the cornea."

Moreover, the "correction" adopted in this invention include, for example, (i) a correction adopted by means of substituting a target one of the measured positions of the ring images by another measured position located nearest the virtual position corresponding to the target one of the measured positions of the ring images, and (ii) a correction adopted by means of substituting the target one of the measured positions of the ring images by the corresponding virtual position of the ring image, however, these corrections (i) and (ii) may be selectively adopted. More specifically, when making judgment, based on the virtual position of the corresponding ring image, as to whether the measured position of the ring image exists within a prescribed range, it is possible to select and adopt the above correction (i) if such measured position exists, or to select and adopt the above correction (ii) if it does not exist.

In preferred form of this invention, upon determining the corneal curvature based on the measured positions of the ring images, a final decision can be made on whether or not to adopt results of the above correction. More specifically, such judgment can be made by adopting an average value of separation distances at several locations between said ring images adjacent to each other after the above correction as a standard separation distance, and by judging whether or not to adopt results of the correction, provided that a difference between the separation distance of the ring images adjacent to each other after the above correction and the standard separation distance is smaller than that between a separation distance of the ring images adjacent to each other at the measured positions of the above ring images and said standard separation distance.

Meanwhile, another aspect of the present invention provides a corneal topography measurement apparatus characterized by comprising: (a) means for obtaining measured positions whereby measured positions of ring images on a cornea are detected by projecting and imaging a plurality of concentric ring lights on the cornea; (b) means for obtaining virtual positions whereby a cross-sectional image of the cornea is taken by irradiating slit lights onto the above cornea, and virtual positions of the ring images obtained by projecting the plurality of ring lights onto the cornea are determined on a basis of said cross-sectional image; (c) means for judging necessity for corrections whereby judgment of a necessity for any correction to the measured positions of the ring images taken by the means for obtaining measured positions is made based on a prescribed correction criteria; (d) means for correction whereby the measured positions of the ring images detected by the means for obtaining measured positions are corrected based on the virtual positions of the ring images taken by the means for obtaining virtual positions contingent upon the judgment made by the means for judging necessity for corrections that such correction is necessary; and (e) means for surface topography calculation whereby surface topography of the above cornea is determined in consideration of results of the correction made by the means for correction based on the measured positions of the above ring images obtained by the means for obtaining measured positions.

Using a corneal topography measurement apparatus equipped with these structures (a) through (e) makes it possible to advantageously implement the above corneal topography measurement method according to this invention. Also, as to the above corneal topography measurement apparatus deemed to be equipped with the structures according to this invention, any of the first through sixth preferred modes described below can be favorably adopted in combination with others as needed and appropriate.

### [First Preferred Mode]

The corneal topography measurement apparatus of the present invention, wherein the means for correction comprises (f) means for judging presence or absence of the measured positions of the above ring images based on the virtual positions thereof within a prescribed allowable range, (g) first means for substitution and adoption whereby the measured position of said ring image located nearest the virtual position of the above ring image is substituted and adopted contingent upon the judgment by said means for judging presence or absence that the measured position of the above ring image exists, and (h) second means for substitution and adoption whereby the virtual position of the above ring image is substituted and adopted contingent upon the judgment by said means for judging presence or absence that the measured position of the above ring image does not exist.

According to such first preferred mode, a correction to an error in relating a ring image to a ring light, for example, can be made with high precision by substituting and adopting the measured position of the ring image located nearest the virtual position thereof. In addition, even in case where the measured position of the ring image has virtually disappeared under the influence of, for example, tear film and ambient light and the like, it becomes possible to avoid data loss at said location reducing the occurrence of errors to a minimum by adopting the virtual position of the ring image.

### [Second Preferred Mode]

The corneal topography measurement apparatus of the present invention further comprises: (i) means for standard separation distance calculation whereby a standard separation distance, which is an average value of separation distances at several locations between the ring images adjacent to each other, is determined after the correction by the means for correction; (j) means for separation distance calculation at measured positions whereby a difference is determined between the separation distance of the ring images adjacent to each other at the measured positions of the ring images and the standard separation distance; (k) means for separation distance calculation at corrected positions whereby a difference is determined between the separation distance at the measured positions of the ring images adjacent to each other after the correction by the means for correction and the standard separation distance; and (1) means for judging whether or not to adopt correction results whereby the measured position of the ring images after the correction by the means for correction is adopted provided that the difference determined by the means for separation distance calculation at corrected positions is smaller than the difference determined by the means for separation distance calculation at measured positions.

According to such second preferred mode, it is possible to effectively and easily verify the correction results and prevent errors caused by unnecessary corrections from occurring by means of objectively evaluating the validity of the measured positions of the ring image. In determining a standard separation distance using the means for standard separation distance calculation, there is no need for referring to the separation distance of all other ring images after correction, and it is enough to refer to at least two other separation distances and average them to obtain a standard separation distance.

### [Third Preferred Mode]

The corneal topography measurement apparatus of the present invention further comprises: display means for displaying surface topography of the cornea determined by the means for surface topography calculation whereby a curvature distribution on a cornea is displayed by color coding corresponding to a curvature.

Adopting the display means of such third preferred mode makes it possible to easily capture corneal topography (curvature) obtained with high precision in accordance with this invention as curvature distribution on the cornea.

### [Fourth Preferred Mode]

The corneal topography measurement apparatus of the present invention, wherein the display means of the above third preferred mode includes: identification display means whereby an area where surface topography of the cornea is determined on a basis of the measured positions of the ring images not corrected by the above means for correction and an area where the surface topography thereof is determined on a basis of the same corrected by the above means for correction are identifiably displayed.

According to such fourth preferred mode, it is now possible to objectively and easily capture the reliability of measured positions of the ring image, and even the reliability of corneal topography displayed by the display means.

### [Fifth Preferred Mode]

The corneal topography measurement apparatus of the present invention, wherein the display means of the above third or fourth preferred mode further includes: peripheral area display means whereby the curvature distribution on the cornea obtained on a basis of the cross-sectional image of the cornea taken by the means for obtaining virtual positions in an outer periphery beyond an area where the ring image is detected by the means for obtaining measured positions is displayed.

According to such fifth preferred mode, it is now possible to display curvature distribution in the peripheral area of the cornea by simple processing based on the virtual position of the ring image. However, curvature distribution in the peripheral area of the cornea has less practical importance than that in the central area, although it makes it possible to efficiently extend the measurement area since curvature distribution is displayed across a wide area while avoiding any increase in the apparatus size and complication of calculation process, which effectively serves as reference and the like in understanding corneal topography in the peripheral area.

### [Sixth Preferred Mode ]

The corneal topography measurement apparatus of the present invention, further comprises at least one of means for displaying virtual ring images whereby the ring image determined by the means for obtaining virtual positions is displayed, and means for displaying corrected ring images whereby the ring image corrected by the means for correction is displayed, in addition to means for displaying measured ring images whereby the ring image taken by the means for obtaining measured positions is displayed.

According to such sixth preferred mode, it is now possible to capture both the measured position and the virtual position or corrected position based on said virtual position by comparing to each other. This makes it possible to simply and easily evaluate, as a process of comparing ring images with each other, the reliability of measured positions, and even the reliability of corneal topography (curvature) obtained.

### [Effects of Invention]

According to the corneal measurement method of this invention, any significant error can be prevented from occurring in determining corneal topography (curvature) based on the measured position of the ring image by means of making corrections based on the virtual position thereof determined on a basis of the cross-sectional image of the cornea, while basically adopting the measured position of the ring image which has high measurement precision, and therefore, it could be made possible to obtain the target corneal topography with insured high precision and reliability.

Also, the corneal measurement apparatus deemed to have a structure according to this invention makes it possible to effectively implement the corneal measurement method of this invention.

### BRIEF DESCRIPTION OF THE DRAWINGS

The foregoing and/or other objects features and advantages of the invention will become more apparent from the following description of a preferred embodiment with reference to the accompanying drawings in which like reference numerals designate like elements and wherein:
FIG. 1 is a cross-sectional model diagram for explaining advantages for a corneal measurement method of this invention to basically adopt measured positions of ring images;
FIG. 2 is a schematic diagram showing the entire structure of a corneal topography measurement apparatus according to one embodiment of the present invention;
FIG. 3 is a model image explaining Z axis alignment equipped in the corneal topography measurement apparatus shown in FIG. 2;
FIG. 4 is an image illustrating a specific example of a cross-sectional image of the cornea obtained by the corneal cross-section imaging apparatus comprising the corneal topography measurement apparatus shown in FIG. 2;
FIG. 5 is an image illustrating a specific example of an measured ring image obtained by a ring image imaging apparatus comprising the corneal topography measurement apparatus shown in FIG. 2;
FIG. 6 is a flow diagram showing a process wherein data are obtained from the images measured in the measured ring calculation unit in FIG. 2;
FIGS. 7A and 7B are diagrams for explaining detailed processing in Steps S 1 to S2 in FIG. 6;
FIG. 8 is a diagram for explaining detailed processing in Steps S1 to S2 in FIG. 6;
FIG. 9 is a diagram for explaining a process of obtaining virtual ring images in the virtual ring calculation unit in FIG. 2;
FIG. 10 is a flow diagram showing the process of obtaining virtual ring images in the virtual ring calculation unit in FIG. 2;
FIG. 11 is a diagram for explaining detailed processing in Steps T1 to T5 in FIG 10;
FIG 12 is an image showing a specific example of a virtual ring image obtained by the virtual ring calculation unit comprising the corneal topography measurement apparatus in FIG. 2;
FIG 13 is a flow diagram showing the overall processing at the judgment and correction unit and the corneal topography calculation and map processing unit in FIG 2;
FIG 14 is a flow diagram showing detailed processing in Steps A1 to A2 of Process (1) in FIG 13;
FIG. 15 is a flow diagram showing detailed processing in Steps A2 of Process (2) in FIG. 13;
FIG.16 is a flow diagram showing detailed processing in Steps A3 of Process (3) in FIG. 13;
FIG. 17 is a diagram for explaining processing in Steps C1 to C2 in FIG. 15;
FIG. 18 is a diagram for explaining processing in Steps C3 to C5 in FIG. 15;
FIG. 19 is a diagram for explaining processing in Step D2 in FIG 16;
FIG. 20 is a diagram for explaining processing in Steps D3 to D5 in FIG. 16;
FIGS. 21A-21C are specific image examples of a measured ring image, a ring image obtained by merged data after correction, and corneal topography obtained by the ring image data collection unit, judgment and correction processing unit, and corneal topography calculation and map processing unit, respectively, comprising the corneal topography measurement apparatus in FIG. 2.
FIG. 22 is an image illustrating an example of an obscure measured ring image obtained by the ring image imaging apparatus comprising the corneal topography measurement apparatus in FIG. 2.
FIG. 23 is an image illustrating an example of an obscure measured ring image obtained by the ring image imaging apparatus comprising the corneal topography measurement apparatus in FIG. 2.
FIGS. 24A and 24B are images illustrating comparative examples of a measured ring image and corneal topography, respectively, obtained by solely using the data in the corneal topography measurement apparatus in FIG. 2;
FIGS. 25A and 25B are images showing as an embodiment a ring image and corneal topography, respectively, obtained by merged data after correction in the corneal topography measurement apparatus in FIG. 2; and
FIG. 26 is an image illustrating a display example of the corrected area in the ring image shown in FIG. 25A.

### DETAILED DESCRIPTION OF PREFERRED EMBODIMENTS

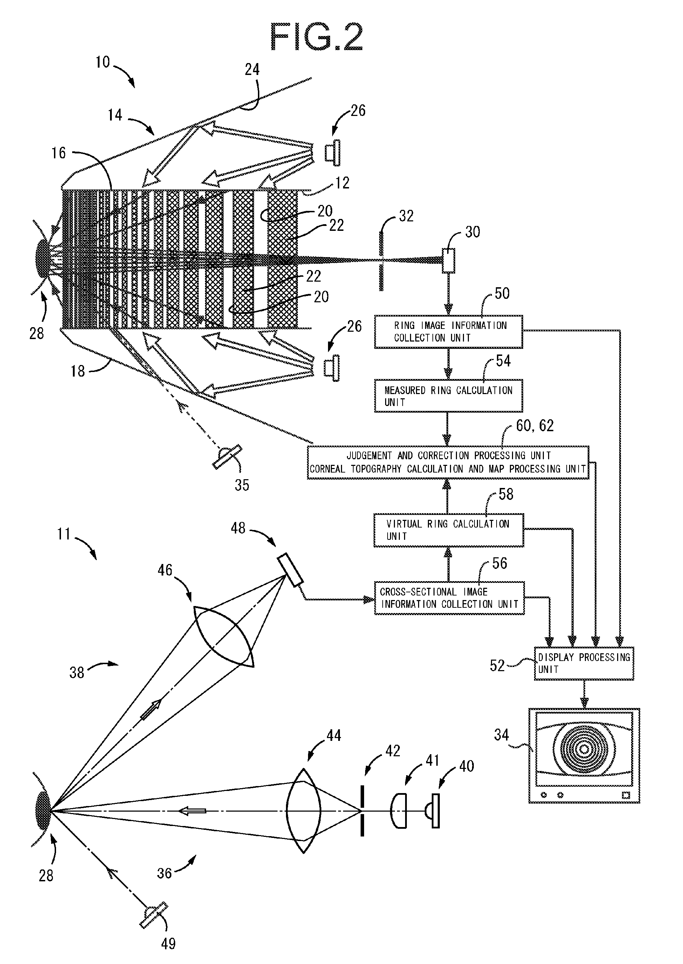

Referring first to FIG 2, there is shown the entire structure of a corneal topography measurement apparatus as one of the embodiments of this invention in a model diagram. This corneal topography measurement apparatus is equipped with both a ring image imaging apparatus 10 for detecting measured positions of the ring image and a corneal cross-section imaging apparatus 11 for obtaining cross-sectional images of the cornea in order to determine virtual positions of the ring image.

The former ring image imaging apparatus 10 is capable of utilizing an imaging apparatus comprising a corneal topography measurement apparatus using a Placido ring that has been publicly known (refer to Patent Literatures 1 and 2). To briefly explain the structure, it is equipped with a cylinder-shaped topocone 14 having a central hole 12 in a straight circular form. The peripheral walls of this topocone 14 constitute a hollow structure comprising an inner wall 16 in a cylindrical form and an outer wall 18 in a tapered form. On the inner wall 16, translucent windows 20 in a form of multiple rings are formed aligned in the axial direction, and the areas other than these translucent windows 20 constitute light shielding parts 22. The outer wall 18 blocks light from outside and its inner side is made to be a reflective plane 24. Toward the rear end of the topocone 14 (right side of FIG.2), LED light sources 26 are installed facing toward the end of the peripheral walls, and the light emitted from these light sources 26 is made to be incident into the peripheral walls as shown by the outline arrows in FIG.2. The light made to be incident into the peripheral walls of the topocone 14 is irradiated into the central hole 12 of the topocone 14 through each of the translucent windows 20 of the inner wall 16. This allows multiple concentric ring lights in a circular form at different angles of incident to be irradiated toward the eye under examination 28 located toward the tip side of the topocone 14 (left side of FIG.2), as shown by solid arrows in FIG.2. In this embodiment, since a total of 34 translucent windows 20 are provided in the topocone 14, a total of 34 ring lights can be irradiated onto the eye under examination 28. In addition, in the area outside the rear end of the topocone 14, a CCD camera 30 and the like is installed along the central axis (Z axis) of the topocone 14 faced against the eye under examination 28. This camera 30 captures, at point blank, projected images of multiple ring lights (ring images) focused on a slit 32 after being irradiated onto and reflected from the cornea of the eye under examination 28, which is displayed in real time on a monitor 34. In this embodiment, the topocone 14 is made of hollow structure, but in case the topocone 14 is made of acrylic resin that has enough translucency, it can be made solid.

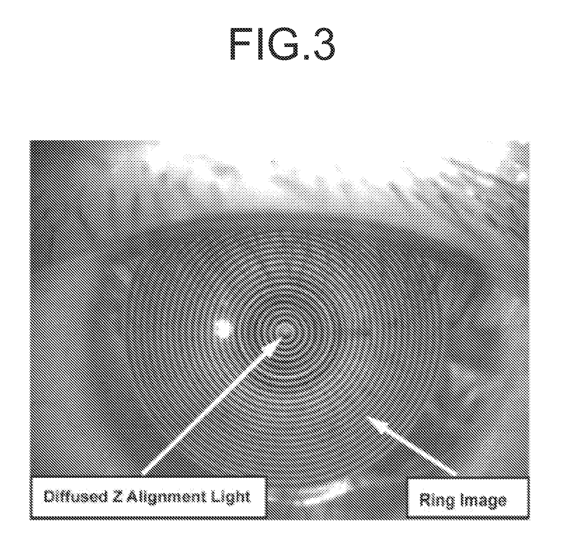

As shown in the model image of FIG.3, an alignment laser 35 for Z axis alignment is also irradiated onto the eye under examination 28, and the incident point of this laser into the eye under examination 28 is captured by the camera 30 and displayed on the monitor 34. The incident point of the alignment laser 35 into the eye under examination 28 is adjusted to locate at the center of the captured image, enabling the adjustment along Z axis against the eye under examination 28. Also, alignments in the directions of X and Y axes that are orthogonal to Z axis can be performed by means of adjusting the location of the cornea of the eye under examination 28 and the ring images looking at those images taken by the camera 30. More specifically, alignments in the directions of X and Y axes as well as that along Z axis can be performed by means of adjusting each center of ring images and the above alignment laser 35 to match the corneal center of the eye under examination 28. The model image of FIG.3 shows a specific example of an image taken by the camera 30 upon completion of the alignment in the ring image imaging apparatus 10 of the present embodiment.

Meanwhile, the corneal cross-section imaging apparatus 11 in FIG.2 may also use a corneal cross-section imaging apparatus that has been publicly known such as the one described in, for example, Japanese Published Unexamined Application No.JP-A-63-197432. To briefly explain the structure, the corneal cross-section imaging apparatus 11 is equipped with a slit projection optical system 36 facing toward the eye under examination 28 along the optical axis and an imaging optical system 38 tilted from the optical axis of the eye under examination 28. The slit projection optical system 36 comprises an illumination light source 40, a projector lens 41, a slit 42 and a projector lens 44. A slit luminous flux emitted from the illumination light source 40 such as LED is parallelized by the projector lens 41 and passes through the slit 42 before being collected by the projector lens 44 to irradiate the eye under examination 28. The imaging optical system 38 comprises an objective lens 46 and a CCD 48, and a luminous flux emitted from the slit projection optical system 36 is diffused by the cornea of the eye under examination 28 is focused on the CCD 48 by the objective lens 46 to capture cross-sectional images of the cornea.

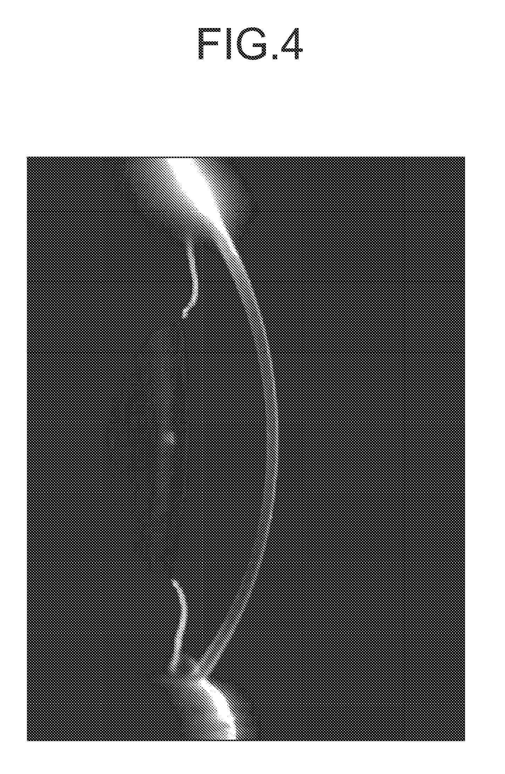

In order to obtain clear cross-sectional images of the cornea by receiving enough light diffused thereby, the central optical axis of the imaging optical system 38 is set at a tilt of 40 to 50 degrees from the central optical axis of the slit projection optical system 36 set on the optical axis of the eye under examination 28. In this case, the main plane of the objective lens 46 and the light receiving plane of the CCD 48 are set to meet Scheimpflug conditions in order to obtain a focused image of the overall corneal cross-sectional image. Also, the corneal cross-section imaging apparatus 11 is made to irradiate a laser beam onto the eye under examination 28 using an alignment laser 49 in the same way as the above ring image imaging apparatus 10 so that it is capable of performing a Z axis alignment by bringing the irradiation point to the corneal center of the eye under examination 28 on the CCD 48 image displayed on the monitor 34. The alignment optical system in the directions of X and Y axes that are orthogonal to Z axis is not expressly shown in the drawings, but can comprise, for example, a light source that projects alignment light on the optical axis of the eye under examination 28 and a light receiving element that receives reflected light thereof. For reference, a specific example of the CCD 48 image upon completion of the alignment is shown in FIG4 for the corneal cross-section imaging apparatus 11 of the present embodiment.

Such corneal cross-section imaging apparatus 11 is made in such a way that the entire system including the slit projection optical system 36 and the imaging optical system 38 cause pivotal displacement around the optical axis of the eye under examination 28 and the slit projection optical system 36. This allows the direction of slit luminous flux eradiated onto the eye under examination 28 in the slit projection optical system 36 and the corneal cross-sectional direction of the eye under examination 28 captured in the imaging optical system 38 to be variable in many steps around the optical axis of the eye under examination 28 so that the images can be taken in multiple radial directions.

Now, the above ring image imaging apparatus 10 and corneal cross-section imaging apparatus 11 may be structurally independent from each other. In that case, each apparatus is provided with an alignment means in each direction of X, Y and Z axes and structured in such a way that the measurement conditions are kept the same for the eye under examination 28. Or, it is also possible to make the ring image imaging apparatus 10 and corneal cross-section imaging apparatus 11 a complex integral structure sharing some optical systems. For example, it is possible to commonalize the light source for X, Y alignments and CCD and the like and use a half mirror and the like to adopt a complex integral structure wherein the ring image imaging apparatus 10 and corneal cross-section imaging apparatus 11 are enabled by selectively switching between them. Also, one can install the ring image imaging apparatus 10 and corneal cross-section imaging apparatus 11 side by side to use them in sequence by selectively sliding them into position in front of the eye under examination.

Then, information is processed by an information processing apparatus equipped with a ring image information collection unit 50 and the like based on shooting data of the ring image taken by the above ring image imaging apparatus 10 as shown in FIG.3 and those of the corneal cross-section taken by the corneal cross-section imaging apparatus 11 as shown in FIG4, thus providing information on the surface topography (curvature) of the cornea of the eye under examination 28. As a result, corneal topography (curvature information) of the targeted eye under examination 28 is displayed on the monitor 34 as map information.

Using the corneal topography measurement apparatus of the present embodiment with the above structure, corneal topography (curvature information) of the eye under examination 28 is measured specifically in the way described below. Details of calculation process performed by the information processing unit of the corneal topography measurement apparatus are described below. This information processing unit comprises a ring image information collection unit 50, a measured ring calculation unit 54, a cross-sectional image information collection unit 56, a virtual ring calculation unit 58, a judgment and correction processing unit 60, and a corneal topography calculation and map processing unit 62, as shown in FIG.2.

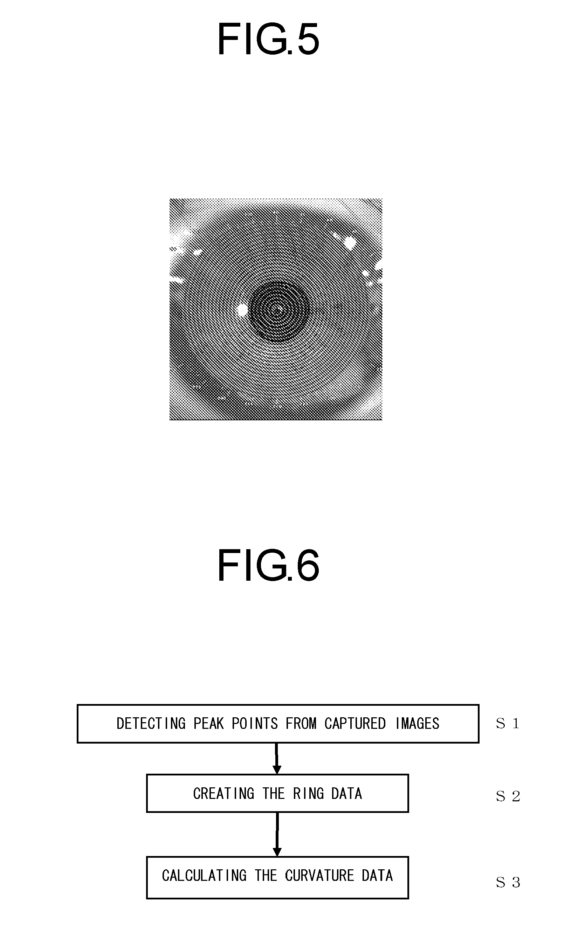

First, information obtained by the camera 30 of the ring image imaging apparatus 10 is converted into image information by undergoing an A/D conversion in the ring image information collection unit 50 as shown in FIG.2, and such image information is sent to the monitor 34 via a display processing unit 52 to be visually displayed thereon (see FIG.5). Such image information is also entered into the measured ring calculation unit 54 to determine each ring location.

In other words, image information of measured ring images obtained from the camera 30 of the ring image imaging apparatus 10 wherein, for example, multiple concentric ring lights are projected on the cornea of the eye under examination as shown in FIG.5 is entered into the measured ring calculation unit 54. By means of processing of Steps S1 through S3 shown in FIG.6 performed by the measured ring calculation unit 54, curvature at multiple measuring points in the area where each target ring light is projected can be determined on a basis of positional information of the measured ring images.

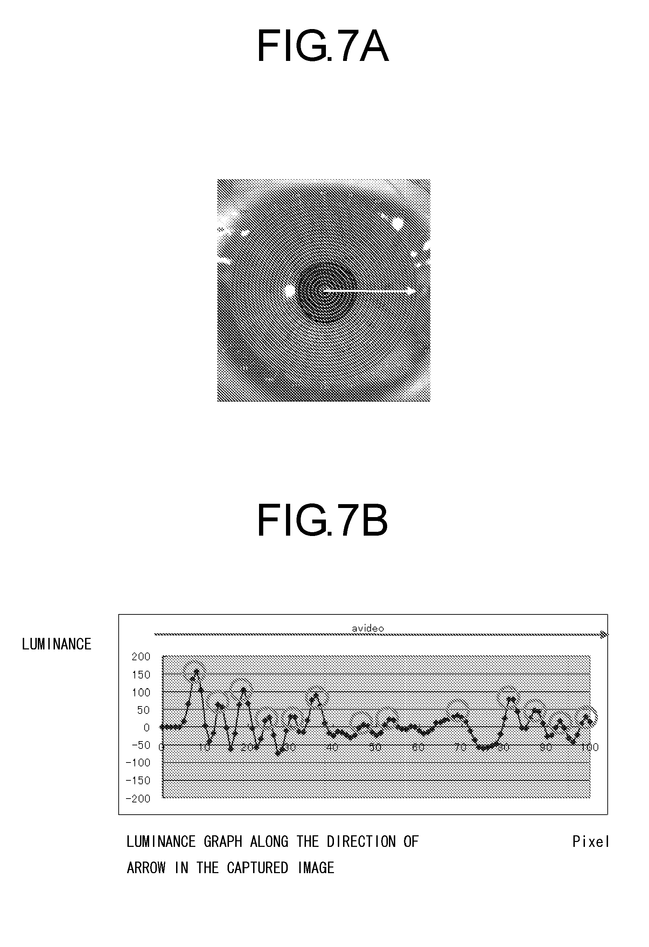

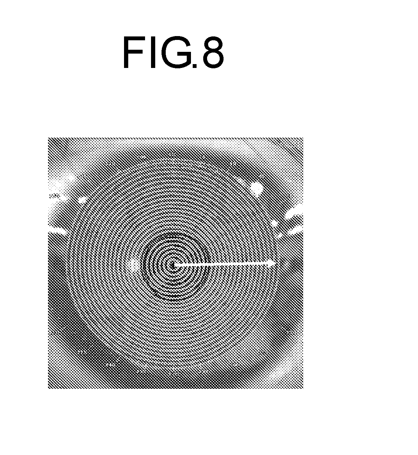

Specifically, information on the ring location is first detected from information on captured images (measured ring images) in Step S1 of FIG6. More specifically, as shown in FIG.7A, a luminance graph is obtained along a particular radial direction orthogonal to the optical axis (for example, the direction shown by the white arrow in FIG.7A) using image information of measured ring images. The horizontal axis of the graph shown in FIG.7B represents separation distance from the optical axis in the radial direction, and the vertical axis represents luminance level. The plotted luminance graph includes multiple peaks, as shown by circles in FIG.7B. These peaks are considered to indicate high luminance points in the captured image, that are, reflection points of concentric ring lights irradiated onto the cornea. Therefore, identifying these positions of luminance peaks allows us to identify each measured ring image in the radial direction along the arrow of FIG.7A. This detection process of peak positions is performed in each radial direction preset around the optical axis. Such measurement points are supposed to be set up at an increment of angle in a circumferential direction around the optical axis, and in the present embodiment, 256 directions are set up in equal interval in a circumferential direction. In summary, in the present embodiment, detection process of ring positions shown in the above FIGS. 7A and 7B takes place in each of 256 directions around the optical axis. Graphic representation as a captured image of each peak point detected this way in all the directions around the optical axis shows a series of almost consecutive points as shown in FIG.8.

Next, in Step S2 of FIG.6, a judgment is made as to which ring light in order of consequence among multiple ring lights projected onto the cornea corresponds to each detected peak point. That is, in a particular radial direction as shown by the white arrow in FIG.8, for example, a ring number is assigned to each peak point from the inner to outer one.

Next, in Step S3 of FIG.6, ring data obtained by a calibration process such as deleting data presumed to be errors due to noise from the ring data obtained in the above Step S2 is adopted, and curvature at each measuring point is determined on a basis of these ring data. As explained above, the ring data of the present embodiment comprise a total of 34 x 256 = 8,704 peak points (measured ring images). In this Step S3, measuring points of curvature are set up at each of a total of 34 x 256 peak points (measured ring images). Since calculation of curvature of corneal surface at each of these measuring points has been known to the public as described in, for example, Japanese Published Unexamined Application No. JP-A-7-124113 and else, and is also beyond the scope of this invention, a detailed description thereof is omitted. However, as shown in the above FIG.1 for example, positions of ring projections onto the corneal surface (A, B) are different depending on the curvature of the corneal surface, and therefore, corneal curvature at said ring positions (measuring points) can be determined by finding the separation distance (r) between the ring position (measuring point) and the optical axis (L), and based on such separation distance, on the assumption that the curvature is in a form of an arc through the corneal apex.

Through the process of the above Steps S1 to S3, curvature at each position of the rings (measuring point) can be determined on a basis of the measured ring image taken by the ring image imaging apparatus 10.

Meanwhile, the virtual ring image and its curvature are determined from the corneal cross-section imaging apparatus 11 shown in FIG.2 in the following way: That is, information obtained by the CCD 48 of the corneal cross-section imaging apparatus 11 is converted into image information by an A/D conversion in the cross-sectional image information collection unit 56, which is in turn sent to the monitor 34 via the display processing unit 52 to visually display the measured cross-sectional image (see FIG.4). Such image information is entered into the virtual ring calculation unit 58 to determine a projected image on the corneal surface obtained on the assumption that each ring light of the ring image imaging apparatus 10 is irradiated onto the corneal surface captured as said corneal cross-sectional image (virtual ring image). Furthermore, the determined virtual ring images are visually displayed, as necessary, by being sent to the monitor 34 via the display processing unit 52. Although what are obtained by the CCD 48 of the corneal cross-section imaging apparatus 11 are corneal cross-sectional images, corneal cross-sectional images at as many positions as tens to hundreds are obtained around said optical axis by means of pivotally displacing the corneal cross-section imaging apparatus 11 around the optical axis of the slit projection optical system 36. In the present embodiment, corneal cross-sectional images are captured in 256 directions around the optical axis, as is the case for measurement by the above ring image imaging apparatus 10. In this way, a total of 256 corneal cross-sectional images are obtained in a form of an approximate arc as shown in FIG.4.

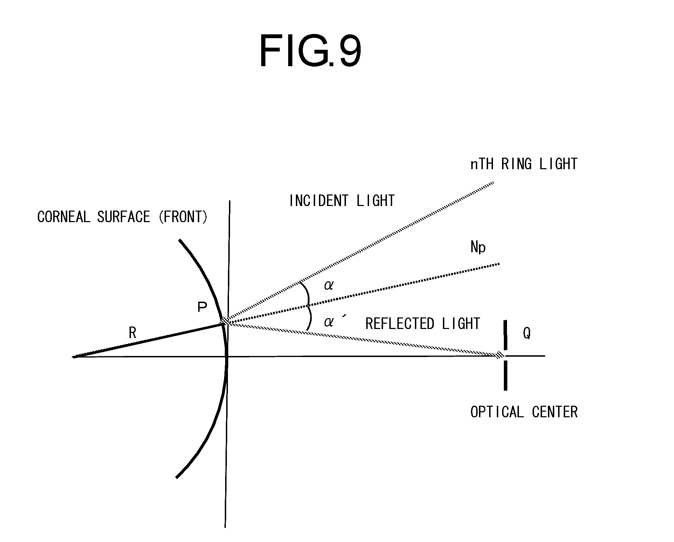

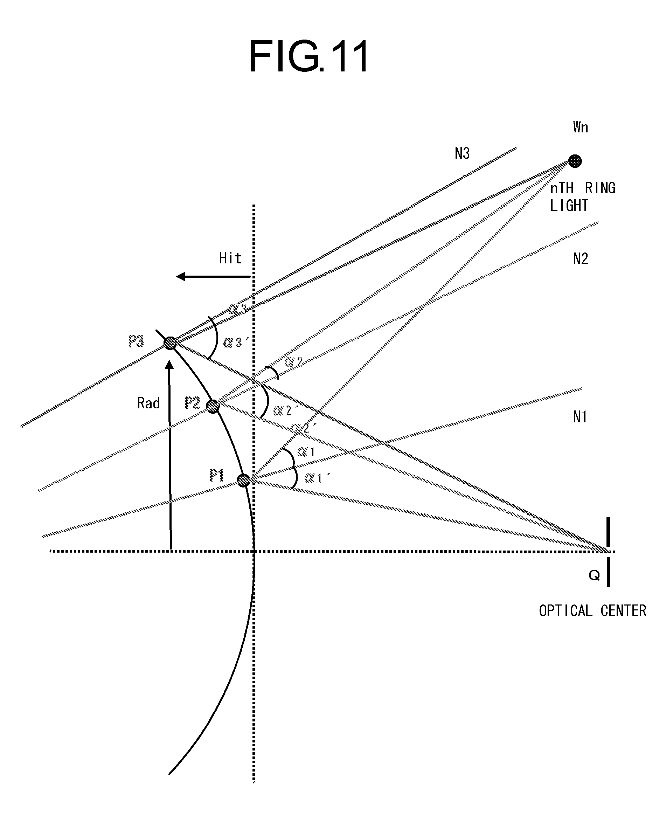

In this section, a principle of determination process of surface curvature and virtual ring image positions performed based on the corneal cross-sectional images in the present embodiment is explained using FIG. 9. First of all, the position of the corneal surface (front) is judged from data such as luminance of the corneal cross-sectional images, and the corneal surface curvature that extends along an arc as shown in FIG.9 is determined. Through this process, curvature at each position on the corneal surface is revealed. Meanwhile, based on this information on the position on the corneal surface, the virtual ring position can be calculated as follows: For easier understanding, a specific example of a case when nth ring light among multiple others appears at point P on the arc created by the corneal surface will be considered. In that case, point P is a reflective point where the light is reflected toward the optical center (aperture). Assuming that the normal at point P on the corneal surface curvature is Nₚ, the incident angle α formed by the light irradiated from the nth ring toward point P and the reflective angle α' from point P toward the optical center Q should be equal on opposite sides of the normal Nₚ. Therefore, if point P is set up at different positions on the corneal surface curvature and the value of angle α formed by the above incident light from the ring toward point P and the normal Nₚ as well as the value of angle α' formed by the normal Nₚ and an imaginary reflected light from point P toward the optical center Q are each determined, an equation α = α' should be satisfied only at the right reflective point, while an inequality α ≠ α' should apply to all other points (see FIG.11). Therefore, if such a point where α = α' is true is found for each of one to n ring lights, the reflective point of each ring light can be determined. In this way, one to n virtual ring images on the cornmeal surface can be determined.

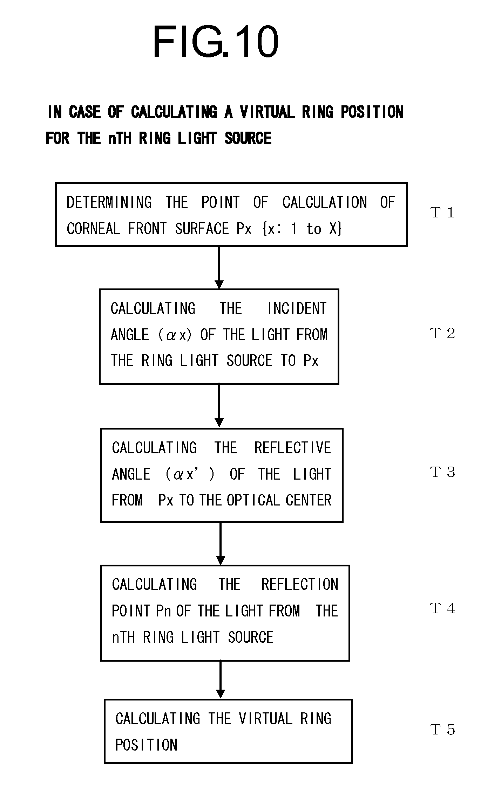

Determination of virtual ring image positions based on such a principle can be made, more specifically, through the process following the Steps T1 through T5 shown in FIG.10. These steps T1 through T5 indicate a flow of determining a virtual ring position for the nth ring light.

First in Step T1, the position of the corneal surface (front) is judged from data such as luminance of corneal cross-sectional images obtained by the CCD 48 of the corneal cross-section imaging apparatus 11 as shown in FIG.4. Through this process, curvature at each position on the corneal surface as shown in FIG.11 is revealed. And, on this corneal surface curvature that extends as an arc, points of calculation Pₓ (x: 1 to X) are set up at multiple locations with equal interval in a circumferential direction as shown in FIG.11. Here, the point of calculation of corneal topography Pₓ is provided with each piece of information on the distance from the center (Rad), height (Hit) and curvature radius (R) based on the coordinate origin located at the intersection between the central optical axis and corneal front surface coordinate, and its coordinate is expressed as Pₓ (Rad, Hit). More specifically, the number of points of calculation Pₓ (the value of X) is generally set within a range of tens to hundreds. Also, the corneal surface curvature at each Pₓ is given by the curvature radius (R).

In the subsequent Step T2, the incident angle at each point of calculation Pₓ of each ring light is calculated for the point of calculation Pₓ (x: 1 to X) found in Step T1 as shown in FIG 11. That is, for each of P1, P2, P3...., Pₓ on the corneal surface curvature, the normal Nₓ and the angle αₓ formed by incident light from the nth ring at each Pₓ are determined. According to the example shown in FIG.11, as the point of calculation progresses from the point nearest the optical axis in the sequence of P₁, P₂, P₃..., the values of α₁, α₂, α₃...gradually decrease. The normal Nₓ is determined from curvature radius R of each point Pₓ.

Moreover, in Step T3, virtual reflective angle αₓ' toward the optical center Q for each calculation point Pₓ (x: 1 to X) is calculated. That is, for each point on the corneal surface curvature P1, P2, P3...., Pₓ, the normal Nₓ and the angle α_{x'} formed by reflected light from point Pₓ to the optical center Q are determined. According to the example shown in FIG.11, as the point of calculation progresses from the point nearest the optical axis in the sequence of P₁, P₂, P₃..., the values of α₁, α₂', α_{3'}...gradually increase.

In the subsequent Step T4, the right reflection point on the corneal surface curvature is found among each of calculation point Pₓ (x: 1 to X) based on each value of αₓ and α_{x'} obtained in the above Steps T2 and T3. That is, among P1, P2, P3...., Pₓ of the nth ring, a point is found where the difference between the above incident angle αₓ and the imaginary reflective angle α_{x'} is the smallest. According to the example shown in FIG.11, the minimum difference between α₁ and α₁ , at point P1 (α₁ ≈ α₁) indicates that the position of P1 is the reflection point of the nth ring.

Also, in Step T5, the position where the reflection point Pₙ found in Step T4 is adjusted in relation to image data of front views in the optical axis direction obtained by the ring image imaging apparatus 10, is calculated. More specifically, such calculation of a virtual ring position can be performed by multiplying the position information of point Pₙ with the camera's compression ratio and unit conversion (pixel) rate.

Such processing of Steps T1 through T5 can be carried out for all of 1 to n ring light sources. In the present embodiment, since 34 ring lights are irradiated by the ring image imaging apparatus 10, the calculation is to be performed for all 34 ring lights in this case, too.

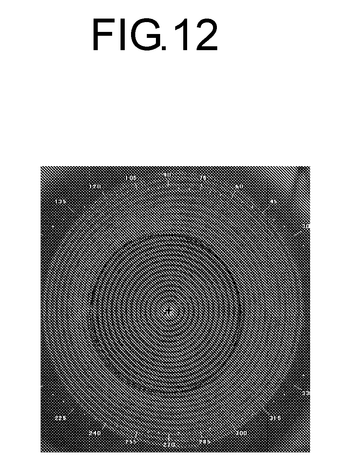

Then, virtual ring positions corresponding to all measuring points set on measured ring images are determined as reflection points Pₙ by means of carrying out the calculation of the above positions Pₙ (n: 1 to 34) on the cornea for all 256 corneal cross-sectional images obtained by the corneal cross-section imaging apparatus 11. That is, corneal cross-sectional images are obtained for all the cross-sections (256 directions in the present embodiment) corresponding to the directions in which peaks and others are measured in the above measured ring images, and reflection points Pₙ (n =1 to 34) are determined on each corneal cross-sectional image, thereby determining a total of 8,704 points as reflection points Pₙ. Then, as shown in FIG.12, a front view of virtual ring images is obtained in the optical axis direction. In summary, these virtual ring images are what are obtained by virtually calculating ring image positions obtained when multiple concentric ring lights are projected by the ring image imaging apparatus 10 onto the corneal topography inferred on a basis of information on the corneal cross-sectional images obtained by the corneal cross-section imaging apparatus 11.

Also, curvature at each measuring point can be determined from information on the corneal surface curvature (curvature radius etc.) used for judgment of positions of the above virtual ring images. These measuring points of curvature are set at 256 points for each of the total 34 ring lights along the circumference totaling at 256 x 34 = 8,704 points in order to correspond to the measuring points for the above measured ring images.

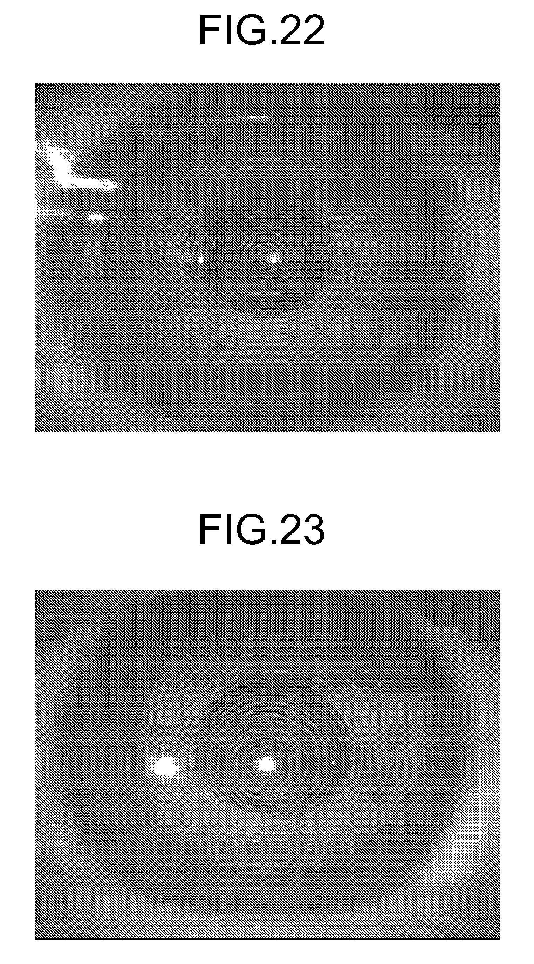

The accuracy of corneal curvature inferred from the cross-sectional images obtained this way by the corneal cross-section imaging apparatus 11 is generally inferior to that of corneal curvature obtained from the measured ring images actually taken by the ring image imaging apparatus 10. As shown in FIG.1, even if the curvature of the corneal surfaces A and B is significantly different, the difference δ of positions on the corneal surface in the corneal cross-section becomes smaller especially near the corneal center, making it difficult to assess accurate curvature. However, virtual ring images created from inference of such cross-sectional images, although its accuracy of curvature is inferior to others, are suitable for capturing the approximate position of each ring image. That is, in case of measured ring images, their reflection points can significantly vary due to diffused reflection from tears that actually cover the cornea and a peculiar shape of the subject's cornea, often causing failure in calculating the corneal curvature (see FIGs.22, 23, 24A, 24B). On the contrary, virtual ring images obtained by calculation from corneal cross-sectional images remain unaffected by tears and others, and continuous ring images can be drawn with no significant variance even for an odd-shaped cornea. Taking advantage of these characteristics, judgment and correction of data for measured ring images can be performed with the data of virtual ring images obtained from the corneal cross-section imaging apparatus 11.

More specifically, as shown in FIG.2, a judgment is made as to whether any correction is necessary to the data of measured ring images through the process wherein the data of measured ring images obtained by the above ring image imaging apparatus 10 and the data of virtual ring images obtained by the above corneal cross-section imaging apparatus 11 are entered respectively into the judgment and correction processing unit 60. Appropriate corrections are to be made, if found necessary, at the judgment and correction processing unit 60 using information on the virtual ring images.

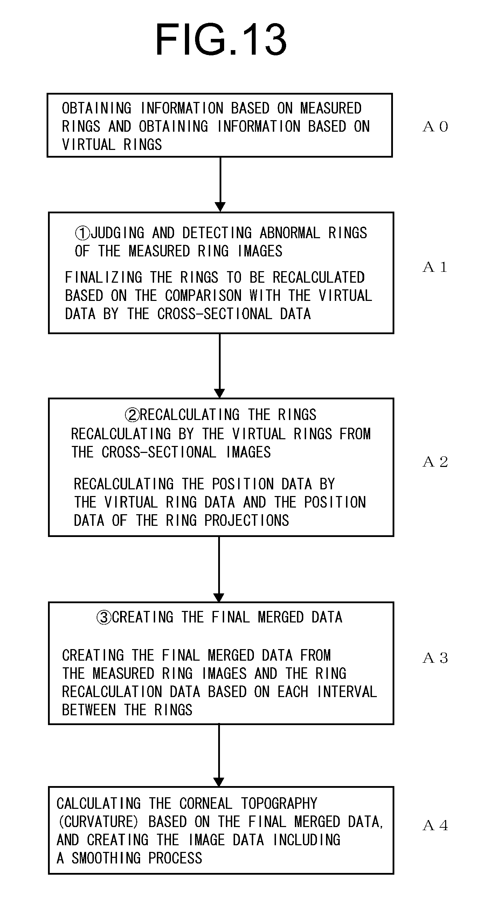

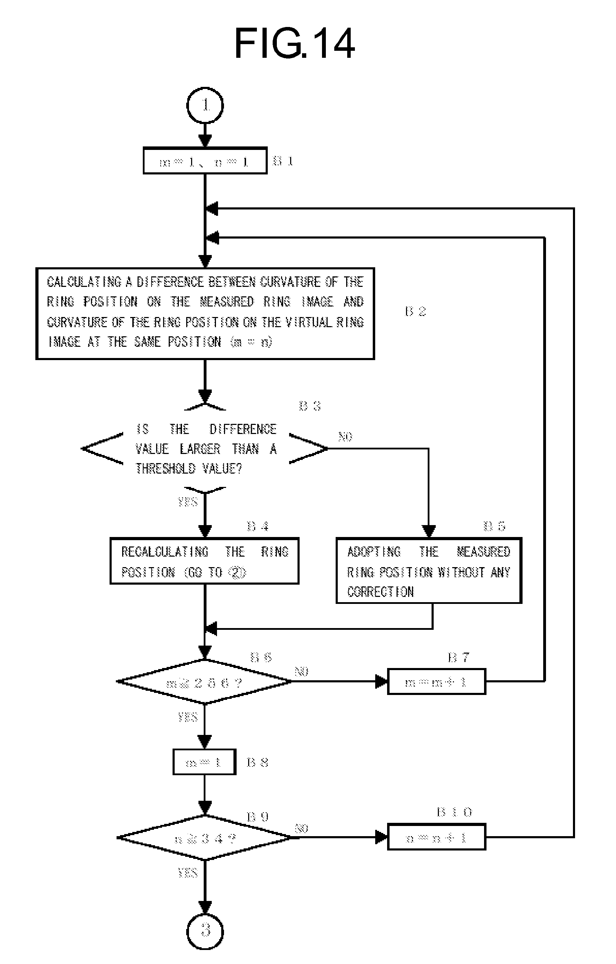

Thus, information (calculation) processing for the purpose of obtaining final information on corrected ring images based on information on measured ring images captured by the ring image imaging apparatus 10 and virtual ring images captured by the above corneal cross-section imaging apparatus 11 is carried out following the flow shown in FIG. 13, for example, at the above judgment and correction processing unit 60 and corneal topography calculation and map processing unit 62. This information processing shown in FIG. 13 is carried out, more specifically, in accordance with the flows shown, for example, in FIGs. 14, 15 and 16.

First, as shown in FIG. 13, positional information of ring images and corneal surface curvature determined by the measured ring calculation unit 54 using measured ring images obtained by the ring image imaging apparatus 10 is obtained in Step A0. Also for virtual ring images obtained by the corneal cross-section imaging apparatus 11, corneal surface curvature is obtained in the same way at multiple measuring points set at corresponding positions on each ring.

Thus, after obtaining positional and curvature information for each of measured rings and virtual rings in Step A0 of FIG.13, judgment is made in the following Step A1 about the necessity for any correction to measured positions of the above measured ring images obtained by the ring image imaging apparatus 10 based on prescribed correction criteria, and the positional information of measured rings to be corrected (recalculated) is fmalized. This judgment of necessity for any correction is made based on the comparison of curvature between virtual ring images obtained by the corneal cross-section imaging apparatus 11 and measured ring images, and carried out depending on whether the difference is no more than prescribed correction criteria. This Step A1 is carried out specifically according to Steps B1 through B10 of FIG 14 as explained later.

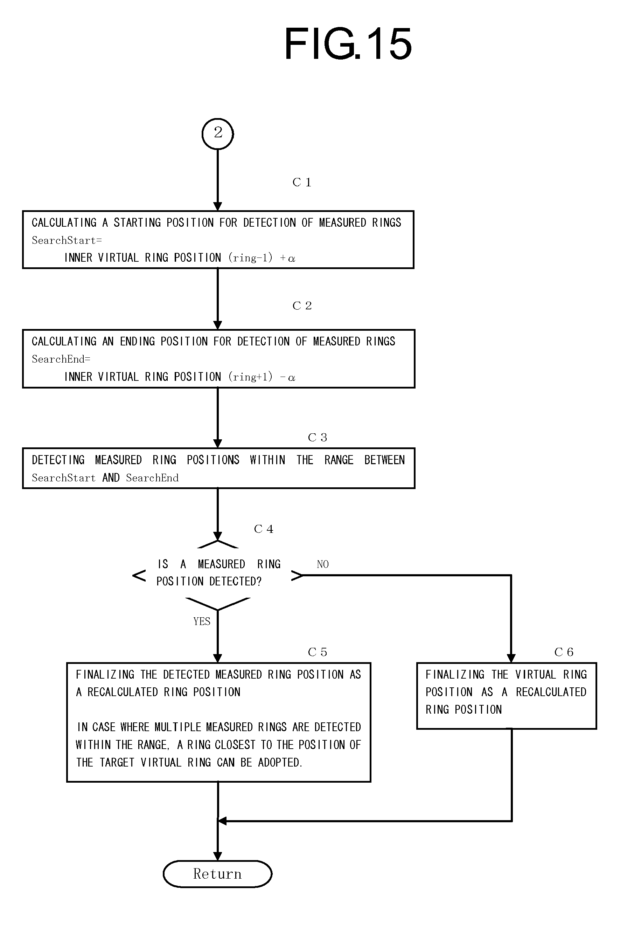

Subsequently, as to measured rings that are judged to require corrections in the above Step A1, corrections to be made is determined on a basis of the positional information of the virtual ring images obtained by the corneal cross-section imaging apparatus 11 and the positional information of said measured rings is recalculated in Step A2 of FIG.13. The correction process for measured rings in Step A2 is carried out specifically in Steps C1 through C6 of FIG.15.

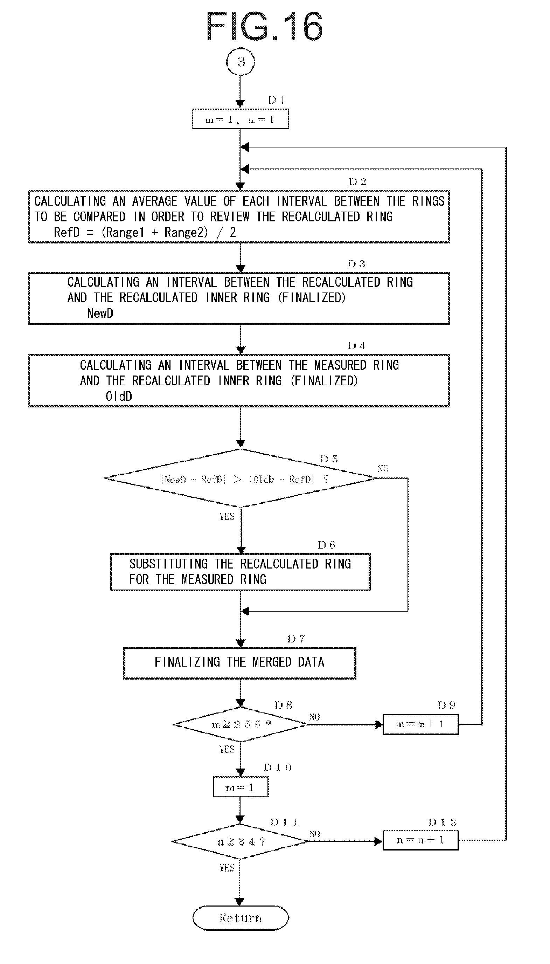

In addition, whether or not to adopt the data of measured rings after corrections obtained in the above Step A2 is verified and determined in Step A3 of FIG. 13. Finally, a final integrated information (final merged data), which is a substantial combination and integration between measured rings and virtual rings, is created by substituting and adopting values recalculated for several measuring points of measured ring images. This process of preparing a final integrated information in Step A3 is carried out specifically in Steps D1 through D12 shown in FIG.16.

Thereafter, a calculation of a target corneal topography (curvature) and an image data creation process including a smoothing process for displaying the calculation results on the monitor 34 are performed in Step A4 of FIG.13 based on the final integrated information determined in the above Step A3. In other words, in this Step A4, the corneal surface topography of the eye under examination 28 is determined on a basis of measured positions of the ring image and by means of finally calculating the corneal surface curvature in consideration of the results of corrections in reference to the virtual ring image. A calculation of corneal surface curvature based on the final merged data of such measured rings can also be performed in accordance with the publicly known calculation process as is the case for calculating curvature based on measured ring images in the above Step S3.

Also, in Steps B1 through B10 (FIG.14) where the above Step A1 (FIG.13) is carried out, the value m that identifies a position on the circumference of the measured ring and the value n that identifies the measured ring are both initialized to 1 first (B1). Here, the value m represents a series of multiple measuring points sequentially coded on a ring that is positioned at equal angle intervals in the circumferential direction per one line in the radial direction, and in the present embodiment, a total of 256 points (m=1 to 256) are set on each ring. The value n represents concentric measured rings sequentially coded from the innermost outward, and in the present embodiment, a total of 34 rings (n= 1 to 34) are set.

Next, at each same position identified by the above values m and n, curvature based on the measured ring image and curvature based on the virtual ring image obtained in the above Step A0 are compared with each other (B2). More specifically, a difference is calculated between curvature by the virtual ring and by measured ring using the corneal surface curvature at the same position as identified by said values m and n. Then, a judgment is made as to whether such difference value is larger or smaller than a prescribed threshold value (B3). If the difference is deemed larger than the threshold value by judgment, the position of the measured ring is corrected by recalculation (B4). On the contrary, if the difference is no more than the threshold value, the measured ring position is adopted as it is without any correction (B5).

Thereafter, the value m is judged, and if said value m is less than the total number of measuring points 256 (B6), 1 is added to the value m before moving to the next measuring point (B7) where a judgment of necessity for corrections and those corrections to all measuring points on the rings are made by means of repeating the above Steps B2 through B7.

Furthermore, with the value m reaching 256 when the processing at all the measuring points on the rings gets completed, the value m is initialized to 1 (B8) before the judgment of the value n, and if said value n has not reached the total number of measured rings 34 (B9), 1 is added to value n before moving to the next measured ring positioned right next to it along the outer circumference (B10). By repeating the above Steps B2 through B10 until the value n reaches 34, the above judgment of necessity for corrections and those corrections to all measuring points on the rings are made for all measured rings.

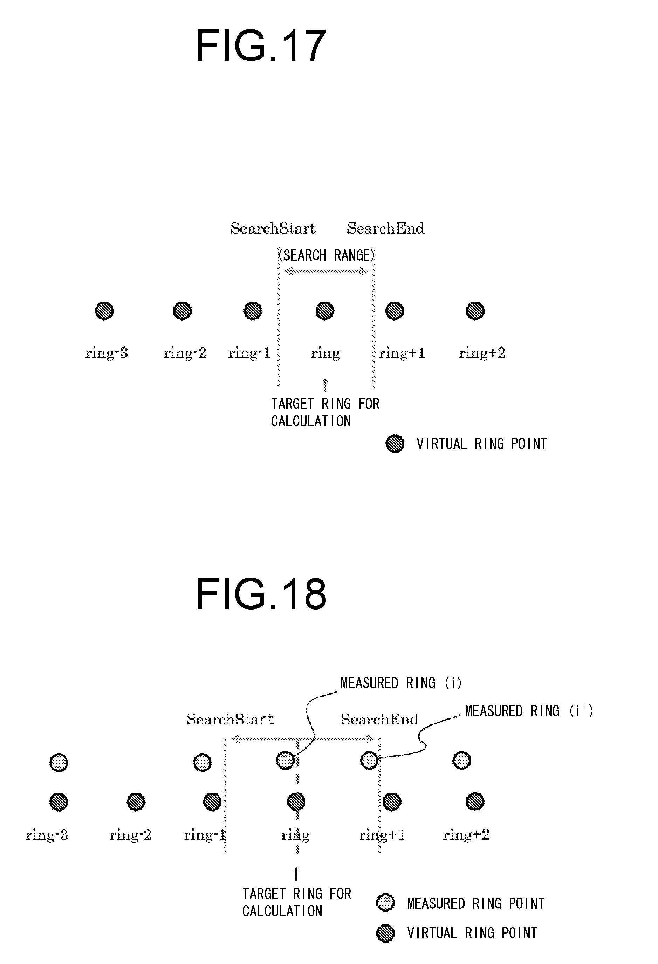

Now, corrections in the above Step B4 are processed in the way shown, for example, in FIG.15. First, in Steps C1 through C4, presence or absence of measured rings inferred to be correct within a specific detection range is confirmed. That is, while a starting position (SearchStart) for detection of measured rings is set in Step C1, an ending position (SearchEnd) for the same is set in Step C2 at the same time. More specifically, as shown in FIG.17, the starting and ending positions within the detection range are set by specifying a given width on each side along the radial direction (inner and outer circumferential sides) for a target ring (for calculation) using the virtual ring information. This range of detection is set within a range short of the adjacent ring on one side ("ring-1") and the one on the other side ("ring +1") for the target virtual ring ("ring"). That is, the value of α in the formulae below is set at less than the distance between adjacent rings. Starting point of detection range (SearchStart) = Position of (ring -1) + α Ending point of detection range (SearchEnd) = Position of (ring +1)―α

Next, in Step C3, presence or absence of rings within an area between the above starting and ending positions of the detection range is confirmed regarding information on measured ring images. More specifically, luminance peaks identifiable as those of rings in the luminance graph of the measured rings shown in FIG.7B are detected within such area.

Thereafter, in Step C4, a judgment is made as to whether a measured ring is detected within the above detection range, and if that is the case, said detected ring is finalized as a recalculated ring by substituting the position data of the above targeted measured ring with those of said detected ring in Step C5. In case where multiple measured rings are detected within the above detection range, a ring closest to the position of the target virtual ring (m, n) can be adopted. More specifically, under the condition shown in FIG 18, for example, if two measured rings (i) and (ii) are detected within the detection range for the target virtual ring ("ring"), the measured ring (i) which is closer to the virtual ring is to be selected. For example, if the measured ring (ii) is related to the target virtual ring ("ring") according to the information on measured rings obtained in Step A0 of the above FIG. 13, information on the position of this measured ring (ii) is substituted with information on the measured ring (i). Meanwhile, if none of the measured rings are detected within such a detection range, the value of the target virtual ring (m, n) is substituted for the position data of the measured ring and adopted as those of a recalculated ring in Step C6.

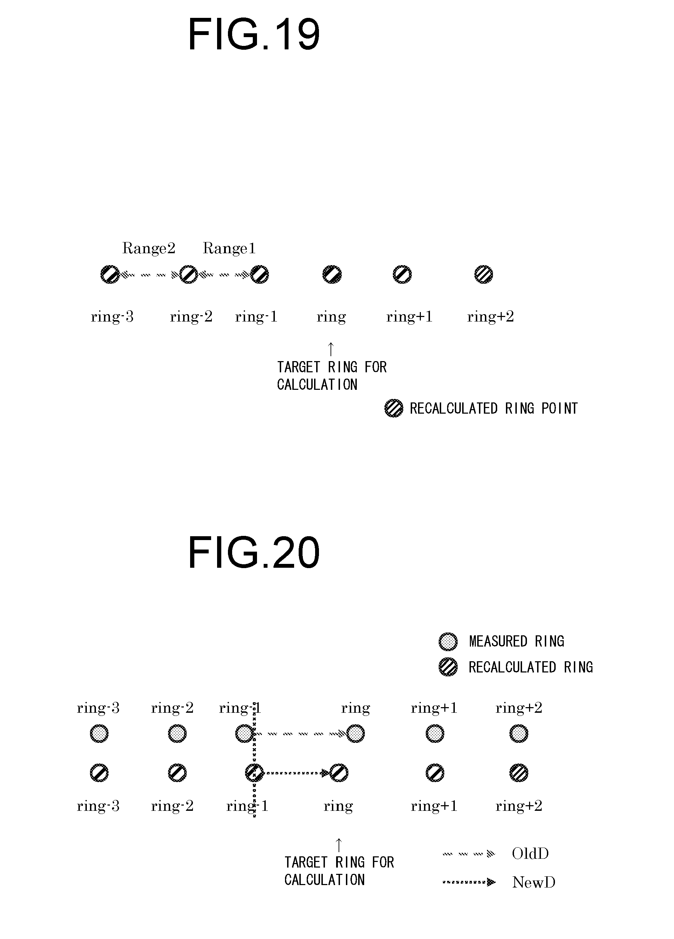

Moreover, in order to create the final integrated information (final merged data) in the subsequent Step A3 after determining the recalculated ring in the way mentioned above by recalculating measured ring images in Step A2 of FIG. 13, the value m identifying a position on the ring circumference and the value n identifying the ring itself are both initialized to 1 (D1), as shown in FIG.16. Then, as shown in FIG. 19, focusing on the recalculated ring at the calculation point identified by the values of m and n ("ring"), an average value (RefD) of each interval (Range1, Range2) between the three rings (ring-1, ring-2, ring-3) located on the inner circumferential side is determined as a standard separation distance (D2). An average value of three or more intervals between the rings can also be adopted as such an average. Besides, in case of setting a calculation point on the innermost circumference where no rings are found to take an average on further inner side, it is also possible to obtain an average value of intervals between the rings for those located on the outer circumferential side of the calculation point.

Meanwhile, at the calculation point ("ring"), an interval (NewD) from the recalculated ring to its inner adjacent ring (ring-1) is determined (D3). Also at the measured ring obtained in Step A0 of FIG 13, an interval (OldD) between the ring corresponding to the calculation point of the recalculated ring ("ring") and its inner adjacent ring (ring-1) is determined (D4). That is, the interval between the recalculated rings determined in Step D3 (NewD) and that between the measured rings (OldD) determined in Step D4 turn out to have a relation of values as shown in FIG.20.

Then, the interval between these recalculated rings (NewD) and that between the measured rings (OldD) are respectively compared to the average of intervals between the rings (RefD), which is a standard separation distance between recalculated rings determined in Step D2 (D5). As a result of such comparison, rings that cause the interval between them to have a smaller difference from the average of intervals are adopted as positional information of said calculation point, which is fmalized and adopted as merged data (integrated information) (D6-D8). In other words, if the interval between measured rings has a smaller difference from the average of intervals between the rings than that between recalculated rings (No in Step D5), positional information on measured rings is adopted as the ring position of said targeted point, whereas if the interval between recalculated rings has a smaller difference from said average than that between measured rings, (Yes in Steps D5), positional information on recalculated rings is adopted as the ring position. (D6).

Thereafter, the value m is judged in Step D9, and merged data for all measuring points on each ring are finalized and adopted by repeating the above Steps D2 through D8 until said value m reaches 256, the total number of measuring points on each ring. In addition, after reaching 256, said value m is initialized before the value n is judged in the subsequent Step 11 (D10), and merged data for all measuring points on each ring are finalized and adopted consecutively, as mentioned above, for all measured rings by repeating the above Steps D2 through D11 until said value n reaches 34, the total number of measured rings (D12).

Once the merged data in Step A3 of FIG. 13 are created in the above way, the corneal topography (curvature) is calculated based on the finally obtained information on merged data in the consequent Step A4, and information on curvature at multiple points (8,704 points) is obtained as map information. Moreover, after a smoothing process necessary for graphically displaying the results, the information is sent to the monitor 34 via the display processing unit 52 and graphically displayed on the monitor 34 superimposed, for example, over the corneal image captured by the camera 30 of the ring image imaging apparatus 10.

As evident in the above description, in the present embodiment, "means for obtaining measured positions whereby measured positions of ring images are detected" includes the ring image imaging apparatus 10 consisting of an information processing apparatus that carries out Step S2 of FIG 6 (more precisely, information processing apparatus that carries out the steps in FIG.6). Also, "means for obtaining virtual positions whereby virtual positions of ring images are determined" includes the corneal cross-section imaging apparatus 11 and consists of an information processing apparatus that carries out Step T5 of FIG. 10 (more precisely, information processing apparatus that carries out the steps in FIG 10). Also, "means for judging necessity for corrections" comprises an information processing apparatus that carries out Step A1 of FIG.13 (more precisely, information processing apparatus that carries out the steps in FIG.14). Additionally, "means for correction whereby the measured positions of the ring images are corrected based on its virtual positions" comprises an information processing apparatus that carries out Step A2 of FIG. 13 (more precisely, information processing apparatus that carries out the steps in FIG.15). Furthermore, such means for correction comprises: "means for judging presence or absence of the measured positions based on the virtual positions of the ring images" and "first means for substitution and adoption whereby the measured position located nearest the virtual position of the ring image is substituted and adopted contingent upon the existence of the measured position" and "second means for substitution and adoption whereby the inferred position of the ring image is substituted and adopted contingent upon the non-existence of the measured position" consisting of the information processing apparatus that carries out the processing shown by Steps C4 though C6 of FIG.15. Moreover, including "means for standard separation distance calculation that determines a standard separation distance which is an average value of separation distances at several locations between the ring images adjacent to each other after the correction by the means for correction" and "means for separation distance calculation at measured positions that determines a difference between the separation distance at the measured positions of the ring images and the standard separation distance" and "means for separation distance calculation at corrected positions that determines a difference between the separation distance at the measured positions after the correction by the means for correction and the standard separation distance," "means for judging whether or not to adopt correction results whereby the final merged data are determined by integrating the measured and corrected positions" comprises the information processing apparatus that carries out Step A3 of FIG. 13 (more precisely, information processing apparatus that carries out the steps in FIG.16). In addition, "means for surface topography calculation whereby surface topography of the cornea is determined in consideration of results of the correction made by the means for correction based on the measured positions of the ring images" comprises the information processing apparatus that carries out Step A4 of FIG.13.

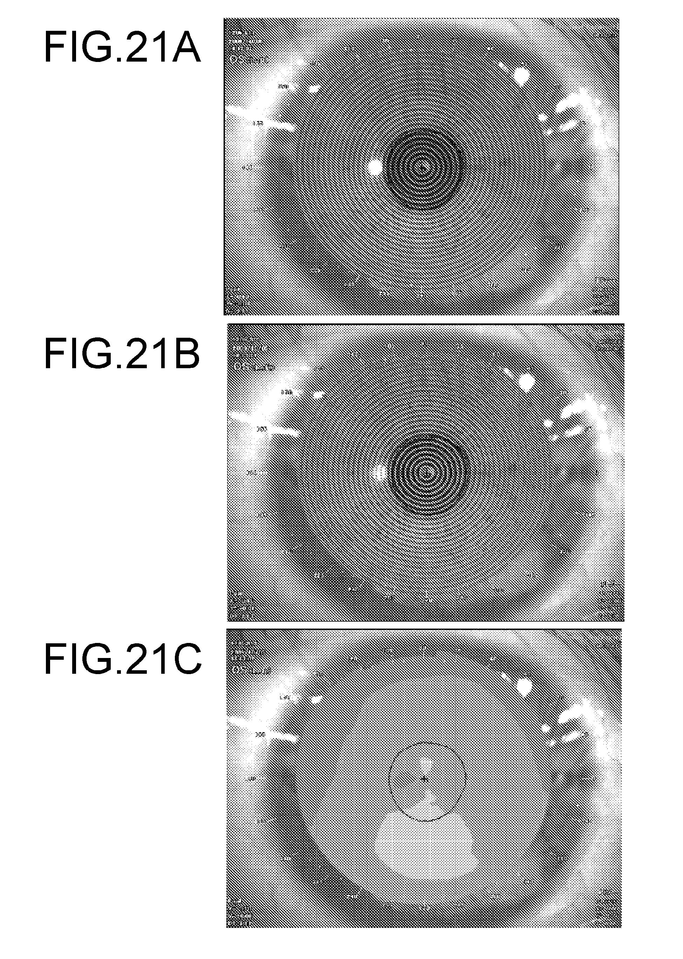

Now, in the present embodiment, measured ring images, for example, obtained in Step A0 of FIG. 13 and sent via the ring image information collection unit 50 of FIG.2 through the display processing unit 52 to be displayed on the monitor 34 can be obtained as shown in FIG.21A. Also, ring images based on the final merged data obtained in Steps A1 through A3 of FIG.13 and sent via the virtual ring calculation unit 58 of FIG.2 through the display processing unit 52 and the judgment and correction processing unit 60 to be displayed on the monitor 34 can be obtained as shown in FIG.21B. As evident from these, means for displaying measured ring images, means for displaying virtual ring images, and means for displaying corrected ring images comprise the monitor 34 in the present embodiment. In addition, corneal topography display images (topomaps) obtained in Step A4 of FIG 13 and sent via the corneal topography calculation and map processing unit 62 of FIG.2 through the display processing unit 52 to be displayed on the monitor 34 can be obtained as shown in FIG21C.

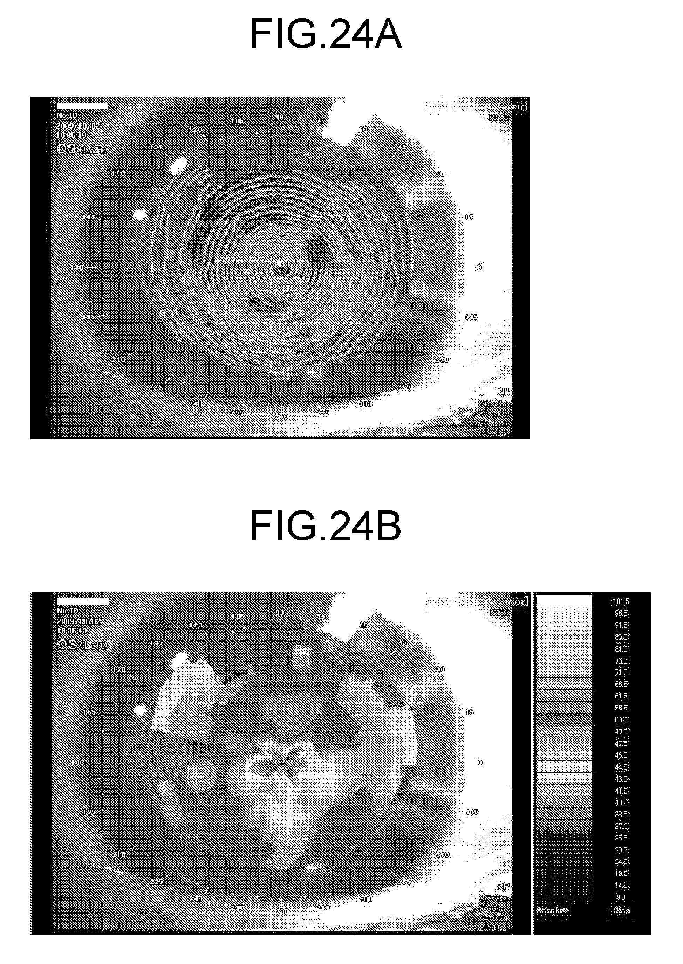

Here, FIGs.21A through 21C show monitor images based on information with almost no error in corneal topography or measurements, but in actual measurements, for example, it is impossible to avoid situations where part of measured rings disappear as shown in FIG.22 due to various thickness of tear film and the existence of ambient light and so forth, or the measured rings partially overlap with each other to be integrated into one as shown in FIG.23. Under these circumstances, if image data are obtained by calculating the corneal topography using only ring information obtained by the ring image information collection unit 50 of FIG.2 and determined by the measured ring calculation unit 54 (i.e. without adopting the virtual ring), information is not fully available with measured rings that have disappeared in many locations on the circumference, as shown in FIG.24A, and therefore, significant errors occur in the corneal topography so that topography beyond reality may be displayed on the monitor display as shown in FIG.24B. In other words, the images displayed in FIGs. 24A and 24B represent a corneal topography measurement apparatus using the Placido ring of conventional structure.

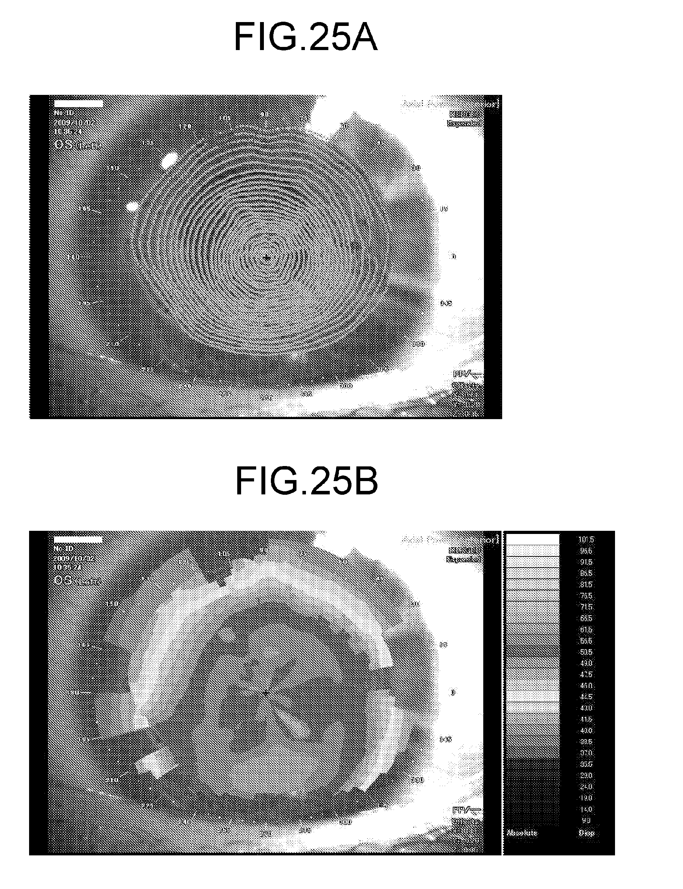

Whereas, if necessary corrections are made to such measured rings as shown in FIG.24A using virtual rings obtained by the corneal cross-section imaging apparatus 11 following the process in Steps A0 through A4 of the above FIG. 13, and the ring image and corneal topography are displayed on the monitor based on the finally obtained merged data, it becomes possible to obtain stable and reliable information as shown in FIGs.25A and 25B.

The reason why a part of the outer circumference is missing in FIG.25B is that the image was graphically displayed as a reference with the corneal curvature determined on a basis of only the corneal surface topography obtained by the corneal cross-section imaging apparatus 11 (i.e. without using any projection information of measured rings) in the outer circumferential area where measured rings are not projected, which was caused by partially missing or defective information on the corneal cross-sectional images. However, it is not essential for this invention to display the corneal surface topography (curvature) for the outer circumferential area where such measured rings are not projected. The indices shown on the right side of FIGs. 24B and 25B are chromatic series that indicate diopter values, and the images of FIGs.24B and 25B are displayed in color for visual observation of diopter value distribution. Thus, the present embodiment allows distribution of curvature on the cornea to be displayed on the monitor 34 with color coding corresponding to the curvature, and this monitor 34 comprises display means. Also, this monitor 34 comprises peripheral area display means whereby distribution of curvature obtained based only on the corneal cross-sectional images in the outer periphery beyond the ring image detection area is displayed.

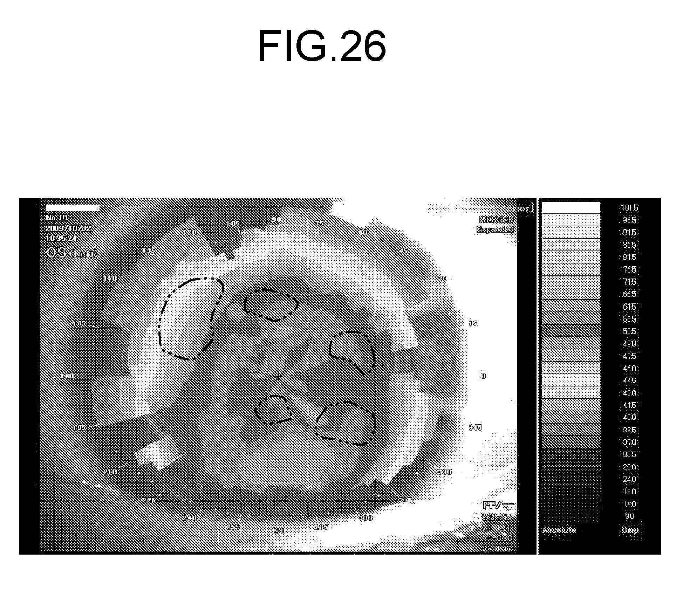

Also, on the image display of corneal topography determined on a basis of merged data as shown in FIG.25B, it is possible to identifiably display the area where corrected values were adopted in substitution for those of virtual rings by means of outlining them and the like as shown in FIG.26. That is, in FIG.26, the area where the corneal surface topography was determined on a basis of measured positions of the ring image without any correction by the virtual ring image as well as the area where the surface topography was corrected using the virtual ring image are displayed on the monitor 34 with distinction by two-dot chain lines, and this monitor 34 comprises identification display means. The image display of corneal topography determined only from information on measured rings shown in FIG.24B and the image display of corneal topography by merged data shown in FIG.25B may be made comparable with each other by parallel or switchable display. In addition to, or in lieu of that, the ring images of FIGs. 24A and 25A can also be displayed in a parallel or switchable way.

The present embodiment of this invention has been described in detail above, but this invention is not to be interpreted as being limited to the above specific descriptions and is implementable, as appropriate, in a modified, corrected or improved form based on the knowledge of those skilled in the art, and it goes without saying that any such form is included within the scope of this invention as long as it does not deviate from the spirit thereof.

## Claims

1. A corneal topography measuring method wherein a plurality of concentric ring lights are projected on a cornea to obtain a plurality of ring images, and a corneal surface topography is measured by obtaining curvatures of the cornea at respective positions where the ring lights are projected based on positional information of the ring images, the method being **characterized by** comprising the following steps:
judging a necessity for any correction to measured positions of the ring images obtained by projecting the ring lights onto the cornea based on a prescribed correction criteria;
determining virtual positions of the ring images based on cross-sectional images of the cornea taken by means of irradiating slit light onto the cornea; and
making a correction to the measured positions of the ring images based on the virtual positions thereof when the correction is deemed necessary to the measured positions of the ring images.

2. The corneal topography measuring method according to claim 1, wherein the step of making the correction selectively executing one of: substituting a target one of the measured positions of the ring images by another measured position located nearest the virtual position corresponding to the target one of the measured positions of the ring images; and substituting the target one of the measured positions of the ring images by the corresponding virtual position of the ring image.

3. The corneal topography measuring method according to claim 1 or 2, further comprising the following steps:
adopting an average value of separation distances at several locations between the ring images adjacent to each other after the correction as a standard separation distance; and
judging whether or not to adopt results of the correction, provided that a difference between the separation distance of the ring images adjacent to each other after the correction and the standard separation distance is smaller than that between a separation distance of the ring images adjacent to each other at the measured positions of the ring images and the standard separation distance.

4. A corneal topography measurement apparatus, **characterized by** comprising:
means for obtaining measured positions whereby measured positions of ring images on a cornea are detected by projecting and imaging a plurality of concentric ring lights on the cornea;
means for obtaining virtual positions whereby a cross-sectional image of the cornea is taken by irradiating slit lights onto the cornea, and virtual positions of the ring images obtained by projecting the plurality of ring lights onto the cornea are determined on a basis of the cross-sectional image;
means for judging necessity for corrections whereby judgment of a necessity for any correction to the measured positions of the ring images taken by the means for obtaining measured positions is made based on a prescribed correction criteria;
means for correction whereby the measured positions of the ring images detected by the means for obtaining measured positions are corrected based on the virtual positions of the ring images taken by the means for obtaining virtual positions contingent upon the judgment made by the means for judging necessity for corrections that such correction is necessary; and
means for surface topography calculation whereby surface topography of the cornea is determined in consideration of results of the correction made by the means for correction based on the measured positions of the ring images obtained by the means for obtaining measured positions.

5. The corneal topography measurement apparatus according to claim 4, wherein the means for correction comprises:
means for judging presence or absence of the measured positions of the ring images based on the virtual positions thereof within a prescribed allowable range;
first means for substitution and adoption whereby the measured position of the ring image located nearest the virtual position of the ring image is substituted and adopted contingent upon the judgment by the means for judging presence or absence that the measured position of the ring image exists; and
second means for substitution and adoption whereby the virtual position of the ring image is substituted and adopted contingent upon the judgment by the means for judging presence or absence that the measured position of the ring image does not exist.

6. The corneal topography measurement apparatus according to claim 4 or 5, further comprising:
means for standard separation distance calculation whereby a standard separation distance, which is an average value of separation distances at several locations between the ring images adjacent to each other, is determined after the correction by the means for correction;
means for separation distance calculation at measured positions whereby a difference is determined between the separation distance of the ring images adjacent to each other at the measured positions of the ring images and the standard separation distance;
means for separation distance calculation at corrected positions whereby a difference is determined between the separation distance at the measured positions of the ring images adjacent to each other after the correction by the means for correction and the standard separation distance; and
means for judging whether or not to adopt correction results whereby the measured position of the ring images after the correction by the means for correction is adopted provided that the difference determined by the means for separation distance calculation at corrected positions is smaller than the difference determined by the means for separation distance calculation at measured positions.

7. The corneal topography measurement apparatus according to any one of claims 4-6, further comprising: display means for displaying surface topography of the cornea determined by the means for surface topography calculation whereby a curvature distribution on a cornea is displayed by color coding corresponding to a curvature.

8. The corneal topography measurement apparatus according to claim 7, wherein the display means includes identification display means whereby an area where surface topography of the cornea is determined on a basis of the measured positions of the ring images not corrected by the means for correction and an area where the surface topography thereof is determined on a basis of the same corrected by the means for correction are identifiably displayed.

9. The corneal topography measurement apparatus according to claim 7 or 8, wherein the display means further includes: peripheral area display means whereby the curvature distribution on the cornea obtained on a basis of the cross-sectional image of the cornea taken by the means for obtaining virtual positions in an outer periphery beyond an area where the ring image is detected by the means for obtaining measured positions is displayed.

10. The corneal topography measurement apparatus according to any one of claims 4-9, further comprising: at least one of means for displaying virtual ring images whereby the ring image determined by the means for obtaining virtual positions is displayed, and means for displaying corrected ring images whereby the ring image corrected by the means for correction is displayed, in addition to means for displaying measured ring images whereby the ring image taken by the means for obtaining measured positions is displayed.
